# EUROPÄISCHE PATENTSCHRIFT

(11) **EP 0 696 910 B1**
(45) Veröffentlichungstag und Bekanntmachung des Hinweises auf die Patenterteilung: **02.11.2000**
(21) Anmeldenummer: 94907473.6
(22) Anmeldetag: 03.03.1994
(51) Int. Cl.: A61F 13/15, A61F 13/62

(54) **GRÖSSENVERSTELLBARES WINDELHÖSCHEN AUS STOFF**
ADJUSTABLE PILCH
COUCHE-CULOTTE EN TISSU A TAILLE REGLABLE

(43) Veröffentlichungstag der Anmeldung: 21.02.1996
(73) Patentinhaber: Ficza, Martin, 3006 Bern (CH)
(72) Erfinder: Ficza, Martin, 3006 Bern (CH)
(74) Vertreter: Schuster, Gregor, Dipl.-Ing.
(86) Internationale Anmeldenummer: CH9400046
(87) Internationale Veröffentlichungsnummer: WO9523569

(56) Entgegenhaltungen:
- WO-A-91/08727
- CA-A- 1 303 796
- CH-A- 684 050
- GB-A- 2 101 875
- GB-A- 2 194 878
- US-A- 3 653 381

## Beschreibung

Es sind waschbare Windelhöschen bekannt, die aber den Nachteil haben, daß mehrere Größen nötig sind, um der zunehmenden Körpergröße des Kindes zu entsprechen. *Um diesen Nachteil zu beseitigen, ist aus der US 3 653 381 ein Windelhöschen bekannt, bei dem das Hinterteil mehrfach umgeklappt werden kann, um die Länge der Windel zu reduzieren. Als nachteilig erweist sich bei diesem bekannten Windelhöschen, daß an dem umklappbaren Hinterteil eine Einlage befestigt ist, die beim Umklappen des Hinterteils ebenfalls umgeschlagen werden muß. Dies führt zu einer großen Dicke des umgeschlagenen Bereichs. Außerdem ist am Vorderteil des Windelhöschens eine Tasche vorgesehen, in die das der Befestigung abgewandte Ende der Einlage eingesteckt wird, um die Einlage zu fixieren.*

Durch den Klettverschluss kann das Höschen dem Bauchumfang entsprechend verschlossen (grössenverstellbar) werden. Dadurch passt das Windelhöschen jedem Kind, ungeachtet von Grösse und Gewicht, optimal. Waschbare Windelhöschen sind den Wegwerfwindeln aus ökologischer und finanzieller Sicht eindeutig vorzuziehen.

*Das Windelhöschen des Anspruch 1 beseitigt die Nachteile des Standes der Technik.*

Im folgenden wird ein Ausführungsbeispiel der Erfindung beschrieben. Es zeigen:

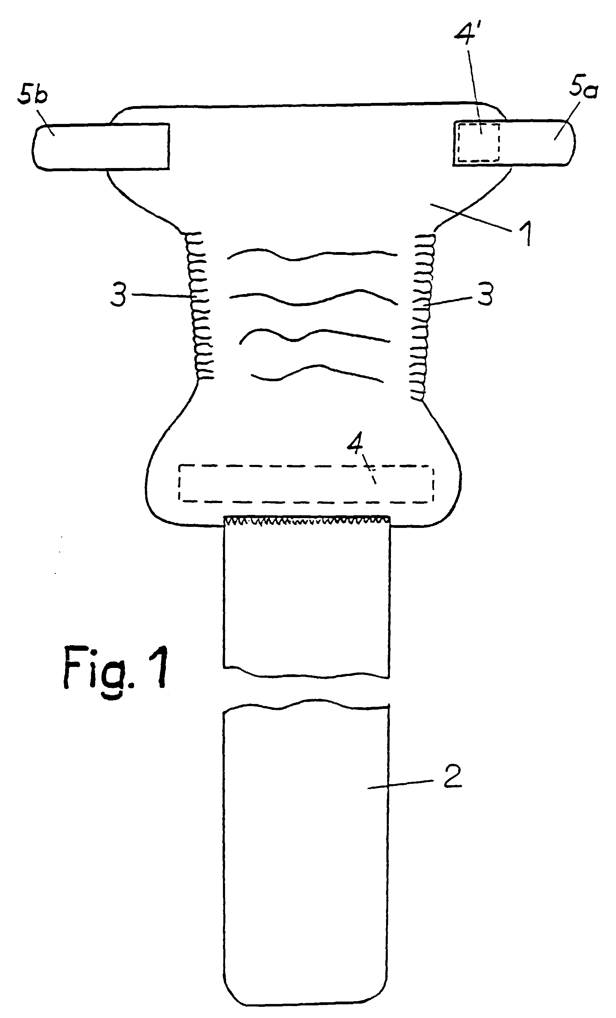

- Figur 1: das Windelhöschen in der Abwicklung
- Figur 2: das Windelhöschen vorbereitet für den Gebrauch für Neugeborene
- Figur 3: das Windelhöschen vorbereitet für den Gebrauch für Kleinkinder

Das Windelhöschen besteht aus einem Höschen Teil 1 und einer am unteren Rand angenähten Einlage 2 aus saugfähigem Material. Für beide Teile wird Baumwolle verwendet, die sehr hautverträglich ist. Die nur einseitige Befestigung der Einlage 2 ermöglicht schnelleres Trocknen der Windel nach dem Waschen. Beim Beinabschluß ist der Rand des Höschenteils 1 mit Gummizügen 3 gerafft, wodurch ein dichter Abschluß um die Beine gewährleistet ist. Der Klettverschluß besteht aus einem Klettband 4 auf der Außenseite des Vorderteils auf der ganzen Breite und Gegenstücke 5a 5b an den Lappen oben rechts und links.

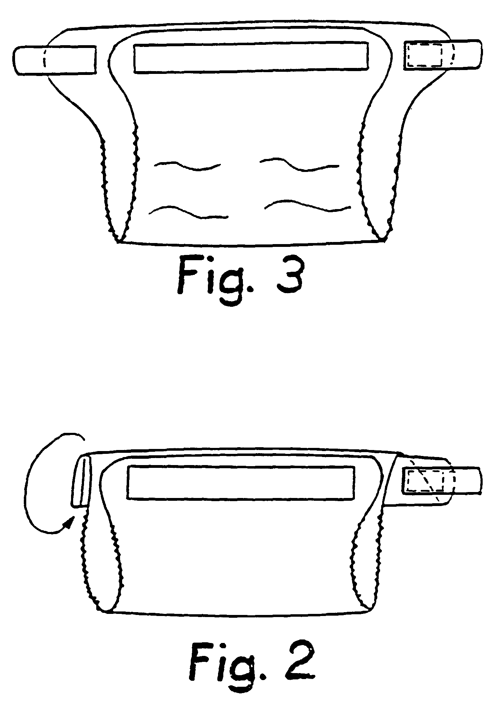

Zum Gebrauch wird die saugfähige Einlage 2 ein- oder zweimal gefaltet eingelegt und der Unterteil samt Einlage nach oben auf den Bauch des Säuglings gelegt. Die Gegenstücke 5a, 5b an den Lappen stimmen dabei mit dem Klettband 4 überein und ermöglichen eine Anpassung an den Bauchumfang des Kindes. Obige Beschreibung bezieht sich auf Fig. 3 für Kleinkinder. Der Gebrauch gemäss Fig. 2 ist für Neugeborene gedacht. In der Fig. 2 ist auf der linken Seite der Lappen mit dem Gegenstück 5b des Klettverschlusses weggelassen, damit das Umlegen besser ersichtlich ist. Das zweimalige Umlegen bringt die Teile des Klettverschlusses wieder in die richtige übereinstimmende Lage. Für den kleinsten Bauchumfang könnte es passieren, dass der Anpassbereich des Klettbandes 4 nicht genügt. Für diesen Fall ist auf der Aussenseite des Gegenstückes 5a ein Klettband 4' befestigt, womit beide Lappen übereinander befestigt werden können.

## Patentansprüche

1. Größenverstellbares Windelhöschen aus Stoff,
mit einem Höschenteil (1),
mit einer einseitig an dem Höschenteil fixierten aus saugfähigem Material bestehenden Einlage (2),
mit einem Beinbereich des Höschenteils (1),
mit einem dem Rücken eines Kindes zugewandten, im Bereich des oberen Randes zur Größenverstellung einrollbaren Hinterteil des Höschenteils,
mit Klettverschlüssen (4, 5) an dem Höschenteil im Bereich des den Bauch eines Kindes umschließenden Vorderteils des Höschenteils und im Bereich des Hinterteils des Höschenteils,
dadurch gekennzeichnet,
daß die Einlage (2) nur an dem Vorderteil befestigt ist, und
daß der Beinbereich jeweils geradlinig ausgebildet ist und über seine gesamte geradlinige Länge einen Gummizug (3) aufweist.

2. Windelhöschen nach Anspruch 1, dadurch gekennzeichnet, dass es aus 100 % Baumwolle gefertigt ist.

3. Windelhöschen nach Anspruch 1, dadurch gekennzeichnet, dass der rechte oder linke Verschlusslappen des Hinterteils auch auf der Aussenseite mit einem Klettverschlussteil (4') versehen ist.

4. Windelhöschen nach Anspruch 3, dadurch gekennzeichnet, dass der Vorderteil auf der Aussenseite, unterhalb des oberen Randes, mit einem Klettstreifen (4 ) durchgehend über die ganze Breite versehen ist.

## Claims

1. Size-adjustable nappy pants made of cloth,
with a pants part (1),
with a pad (2) made of absorbent material fixed on one side of the pants part,
with a leg area on the pants part (1),
with a roll-up rear section of the pants part facing the back of the child, in the area of the top edge, for size adjustment,
with Velcro fastenings (4, 5) on the pants part in the area of the front part of the pants enclosing the stomach of the child and in the area of the rear part of the pants,
characterised in that
the pad (2) is fastened only to the front part, and
the leg area is shaped in a straight line in each case and features an elastic strip (3) over its entire straight length.

2. Nappy pants in accordance with Claim 1, characterised in that they are made of 100% cotton.

3. Nappy pants in accordance with Claim 1, characterised in that the right or left fastener flap of the rear part features a Velcro fastener part (4^{*1*}) also on the outer side.

4. Nappy pants in accordance with Claim 3, characterised in that the front part on the outer side, below the top edge, features a continuous Velcro strip (4) over the entire width.

## Revendications

1. Couche-culotte réglable en taille en tissu,
avec une partie slip (1),
avec une doublure (2) à base de matériau absorbant et fixée d'un côté sur la partie slip,
avec une zone jambe de la partie slip (1)
avec une partie arrière de la partie slip qui est tournée vers le dos d'un enfant et peut être enroulée dans la zone du bord supérieur pour le réglage de la taille,
avec des bandes velcro (4, 5) sur la partie slip dans la zone de la partie avant entourant le ventre d'un enfant et dans la zone de la partie arrière de la partie slip,
caractérisée
en ce que la doublure (2) est fixée ici sur la partie avant, et ce que la zone de la jambe est conçue respectivement en ligne droite et présente une bande élastique (3) sur toute sa longueur rectiligne.

2. Couche-culotte selon la revendication 1, caractérisée en ce qu'elle est fabriquée pour 100% en coton.

3. Couche-culotte selon la revendication 1, caractérisée en ce que la languette de fermeture droite ou gauche de la partie arrière est pourvue d'une partie de bande velcro (4') également sur le côté extérieur.

4. Couche-culotte selon la revendication 3, caractérisée en ce que la partie avant est pourvue sur le côté extérieur, au-dessous de la bande supérieure, d'une bande velcro (4) de façon continue sur toute la largeur.
